# EUROPÄISCHE PATENTSCHRIFT

(11) **EP 3 984 048 B1**
(45) Veröffentlichungstag und Bekanntmachung des Hinweises auf die Patenterteilung: **08.02.2023**
(21) Anmeldenummer: 20740508.5
(22) Anmeldetag: 08.06.2020
(51) Int. Cl.: G21F 9/02, G21F 9/08, G01N 33/00

(54) **VERFAHREN ZUR RADIOLOGISCHEN CHARAKTERISIERUNG DES STRAHLUNGSINVENTARS VON QUECKSILBER**
METHOD FOR THE RADIOLOGICAL CHARACTERISATION OF THE RADIATION LISTING OF MERCURY
PROCÉDÉ PERMETTANT LA CARACTÉRISATION RADIOLOGIQUE DE L'INVENTAIRE DE RAYONNEMENT DE MERCURE

(30) Priorität: 11.06.2019 DE 102019115733
(43) Veröffentlichungstag der Anmeldung: 20.04.2022
(73) Patentinhaber: Argentum Vivum Solutions GmbH, 22335 Hamburg (DE)
(72) Erfinder: FRANCK, Michael, 23569 Lübeck (DE)
(74) Vertreter: Lobemeier, Martin Landolf
(86) Internationale Anmeldenummer: PCT/DE2020/100480
(87) Internationale Veröffentlichungsnummer: WO 2020/249159

(56) Entgegenhaltungen:
- WO-A1-2006/074960
- US-A1- 2017 160 409
- Larissa Klaß ET AL: "Measurement Concept for a Possible Clearance of Mercury Waste from Nuclear Facilities - 19138", WM2019 Conference, 7. März 2019 (2019-03-07), XP055724587, Phoenix, Arizona, USA Gefunden im Internet: URL:http://juser.fz-juelich.de/record/8669 56/files/Main%20document.pdf [gefunden am 2020-08-24]
- THORSTEN BARTELS-RAUSCH ET AL: "Interaction of gaseous elemental mercury with snow surfaces: laboratoryÂ investigation; Interaction of gaseous elemental mercury with snow surfaces: laboratory investigation", ENVIRONMENTAL RESEARCH LETTERS, ENVIRONMENTAL RESEARCH LETTERS, BR, Bd. 3, Nr. 4, 1. Oktober 2008 (2008-10-01) , Seite 45009, XP020148443, ISSN: 1748-9326

## Beschreibung

Die Erfindung betrifft ein Verfahren zur radiologischen Charakterisierung des Strahlungsinventars von Quecksilber. Die Erfindung betrifft auch eine Vorrichtung zur Durchführung des Verfahrens.

Zur radiologischen Charakterisierung des Strahlungsinventars von Quecksilber werden beispielsweise die aus Larissa Klaß et al: "Measurement Concept for a Possible Clearance of Mercury Waste from Nuclear Facilities - 19138", WM2019 Conference, March 3-7, 2019 Phoenix Arizona, USA, aus der US 2017/160409 A1, aus Thorsten Bartels-Rausch et al. "Interaction of gaseous elemental mercury with snow surfaces: laboratory investigation", Environmental Research Letters, BR, Bd. 3, Nr. 4 (2008-10-01), Seite 45009 , ISSN: 1748-9326 und aus der WO 2006/074960 A1 bekannte Verfahren angewendet.

Quecksilber, beispielsweise aus der Verwendung als Dichtungsmaterial in sogenannten Heißen Zellen, also stark abgeschirmten Räumen zur Handhabung und kurzfristigen Lagerung von hochradioaktiven Substanzen, oder aus der Verwendung als Spallationstarget in Beschleunigeranlagen, das mit radioaktiven Stoffen kontaminiert sein kann, muss im Zuge eines Dekontaminations- und/oder Freigabeverfahrens radiologisch charakterisiert werden.

Zur Quantifizierung der im Quecksilber enthaltenen Radionuklide (anderer Elemente) werden zur Charakterisierung der Gesamtmasse üblicherweise gammaspektrometrische Messungen an einzelnen Proben angewendet, wobei hierdurch nicht nachweisbare Radionuklide ergänzend durch zerstörende analytische Verfahren quantifiziert und zur Herleitung geeigneter Nuklidvektoren berücksichtigt werden.

Dieses Vorgehen ist insofern problematisch, dass die untersuchte Probe als repräsentativ gelten muss, um von der Probe Rückschlüsse auf die zu beurteilende Gesamtmenge ziehen zu können. Ein standardisiertes Homogenisierungsverfahren mit reproduzierbaren Ergebnissen für die Probenahme bei Quecksilber ist jedoch nicht bekannt.

Des Weiteren besteht bei der radiologischen Charakterisierung von Quecksilber in Hinblick auf das Strahlungsinventar das Problem, dass die Messung der radioaktiven Strahlung durch die hohe Abschirmung des Quecksilbers gestört wird.

Aufgabe der Erfindung ist es daher, ein Verfahren zu schaffen, dass eine normative radiologische Charakterisierung von Quecksilber ermöglicht. Insbesondere ist es Aufgabe der Erfindung, ein Verfahren zur Überprüfung der Kontamination von Quecksilber mit Radionukliden, insbesondere von Radionukliden anderer Nicht-Quecksilber-Elemente, zu ermöglichen, um in Hinblick auf das Strahlungsinventar geeignete Dekontaminations- und/oder Freigabeverfahren einleiten zu können.

Diese Aufgabe wird erfindungsgemäß durch das Verfahren mit den Merkmalen von Anspruch 1 und die Vorrichtung mit den Merkmalen von Anspruch 8 gelöst. Die Unteransprüche geben jeweils vorteilhafte Ausgestaltungen der Erfindung wieder.

Grundgedanke der Erfindung ist es, gegebenenfalls mit Radionukliden anderer Elemente radioaktiv kontaminiertes Quecksilber in den gasförmigen Aggregatzustand zu überführen, wodurch der Abstand der Quecksilberatome zueinander vergrößert und die Abschirmwirkung des Quecksilbers in Bezug auf die im Quecksilber enthaltenen Radionuklide anderer Elemente verringert wird. Dieses Vorgehen ermöglicht bei Einhaltung entsprechend vorgegebener Parameter, insbesondere Druck und Temperatur, eine standardisierte radiologische Charakterisierung von Quecksilber, ohne dass eine vorherige Aufbereitung, insbesondere Homogenisierung, der Probe notwendig ist. Speziell ermöglicht das Verfahren eine kontinuierliche Messung der gesamten zu charakterisierenden Masse.

Wird speziell die gesamte Menge von beispielsweise als Dichtungsmaterial bei heißen Zellen verwendetem oder aus der Verwendung als Spallationstarget angefallenem Quecksilber hinsichtlich dessen Strahlungsinventars kontinuierlich vermessen, ist eine Fraktionierung des Quecksilbers hinsichtlich der in der entsprechenden Fraktion gemessenen Radioaktivität vorgesehen. Aufgrund dieser Maßnahme ist es bei einer erhöhten Radioaktivitätsmessung nicht erforderlich die Gesamtmenge einer bestimmungsgemäßen Entsorgung zuzuführen, sondern nur die entsprechend mit einer hohen Radioaktivität belastete Fraktion.

Im Gegensatz zu den bekannten Verfahren kann aufgrund der Erfindung eine qualitativ und quantitativ zuverlässige Charakterisierung des Quecksilbers erfolgen.

Anders als beispielsweise bei dem aus der US 2017/0160409 A1 bekannten Verfahren, dass lediglich die Messung einer homogenen Probe eines β-Strahlers durch direktes In-Kontaktbringen der in den gasförmigen Zustand überführten Probe mit einer Vielzahl von Szintillationskörpern verbessert, beseitigt das vorliegende Verfahren den abschirmenden Einfluss des Quecksilbers auf das in diesem enthaltene Inventar an γ-Strahlem, ohne dass eine aufwändige Probenvorbereitung notwendig ist.

Erfindungsgemäß wird also ein Verfahren zur radiologischen Charakterisierung des Strahlungsinventars von Quecksilber vorgeschlagen mit den Schritten: a. Bereitstellen einer vorbestimmten Menge Quecksilber, b. Verdampfen wenigstens einer Teilmenge des bereitgestellten Quecksilbers, und c. Erfassen der vom gasförmigen Quecksilber ausgehenden radioaktiven γ-Strahlung. Bei dem bereitgestellten Quecksilber handelt es sich insbesondere um Dichtungsmaterial aus Heißen Zellen oder um Quecksilber aus der Verwendung als Spallationstarget.

Bevorzugt sind die weiteren Schritte d. Kondensieren des gasförmigen Quecksilbers, e. Auffangen des kondensierten Quecksilbers, und f. Entsorgen des Quecksilbers in Abhängigkeit von der gemessenen radioaktiven γ-Strahlung vorgesehen, wobei das Entsorgen das Aufbereiten des Quecksilbers umfassen kann und entsprechend vorgesehener Regularien erfolgt.

Eine weitere bevorzugte Ausgestaltung sieht vor, dass die bereitgestellte Menge Quecksilber und/oder der für das Verdampfen vorgesehene Teilmenge des bereitgestellten Quecksilbers und/oder das aufgefangene kondensierte Quecksilber gewogen werden, wobei besonders bevorzugt die spezifische Aktivität durch Berechnen der gemessenen radioaktiven γ-Strahlung bezogen auf das Gewicht der bereitgestellten Menge Quecksilber und/oder der für das Verdampfen vorgesehenen Teilmenge des bereitgestellten Quecksilbers und/oder des aufgefangenen kondensierten Quecksilbers bestimmt wird.

Das Verfahren weist speziell das Erfassen der radioaktiven γ-Strahlung durch gammaspektroskopisches Messen der radioaktiven Strahlung und Aufzeichnen eines radioaktiven Strahlungsspektrums auf. Dieses Vorgehen ermöglicht besonders bevorzugt das Bestimmen der im Quecksilber enthaltenen Radionuklide mittels des erfassten Gammaspektrums und deren quantitative Bestimmung mittels gammaspektrometrischer Methoden. Das Entsorgen des Quecksilbers kann dadurch besonders bevorzugt in Abhängigkeit von der gemessenen radioaktiven γ-Strahlung und der Art der bestimmten, im Quecksilber enthaltenen Radionuklide erfolgen.

Ebenso wird erfindungsgemäß eine Vorrichtung zur radiologischen Charakterisierung des Strahlungsinventars von Quecksilber vorgeschlagen, die einen Vorlagebehälter zur Aufnahme einer zu untersuchenden Quecksilberprobe, eine auf den Vorlagebehälter wirkende Heizeinrichtung zum Verdampfen der Quecksilberprobe, eine mit dem Vorlagebehälter verbundene Messstrecke, die ein Gammaspetkrometer zum Erfassen der vom gasförmigen Quecksilber ausgehenden radioaktiven γ-Strahlung aufweist, und einen mit der Messstrecke verbundenen Sammelbehälter zum Sammeln der die Messstrecke durchlaufenen Quecksilberprobe aufweist.

Bevorzugt ist eine auf die Messstrecke wirkende weitere Heizeinrichtung vorgesehen, die das Quecksilber vom Vorlagebehälter in die Messstrecke, beispielsweise eine Rohrleitung, im gasförmigen Zustand hält.

Weiter ist bevorzugt vorgesehen, dass zwischen der Messstrecke und dem Sammelbehälter eine Kühleinrichtung zum Kondensieren des gasförmigen Quecksilbers eingerichtet ist, sodass die Probe in ihren Ursprungszustand überführt werden kann.

Des Weiteren ist bevorzugt eine erste Waage zur Bestimmung der Masse der im Vorlagebehälter befindlichen Quecksilberprobe und/oder eine zweite Waage zur Bestimmung der Masse der im Sammelbehälter befindlichen Quecksilberprobe vorgesehen. Mithilfe der Waagen kann einerseits die spezifische Radioaktivität der Quecksilberprobe bestimmt werden. Andererseits kann die Qualität der Messung bzw. die Sicherheit der Vorrichtung durch Vergleich der Werte der Waagen überprüft werden.

Schließlich ist die Vorrichtung aus Sicherheitsgründen bevorzugt vakuum- und druckdicht ausgeführt.

Die Erfindung wird im Folgenden anhand eines in der beigefügten Zeichnung dargestellten, besonders bevorzugt ausgestalteten Ausführungsbeispiels näher erläutert:

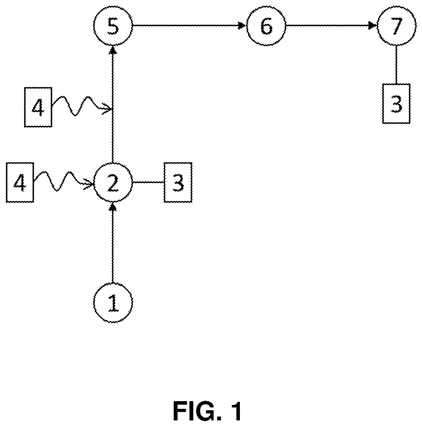

Fig. 1 zeigt den schematischen Aufbau eines besonders bevorzugt ausgestalteten Ausführungsbeispiels einer vakuum- und druckdicht ausgebildeten Vorrichtung zur radiologischen Charakterisierung des Strahlungsinventars von Quecksilber. Die Vorrichtung weist einen Vorratsbehälter 1 auf, aus dem die zu untersuchende Quecksilberprobe in einen Vorlagebehälter 2 überführt wird. Der Vorlagebehälter 2 ist zur Aufnahme der zu untersuchenden Quecksilberprobe eingerichtet und mit einer auf den Vorlagebehälter 2 wirkenden Heizeinrichtung 4 zum Verdampfen der Quecksilberprobe ausgestattet.

An den Vorlagebehälter 2 anschließend ist eine mit dem Vorlagebehälter 2 verbundene Messstrecke 5 vorgesehen, die ein Gammaspektrometer zum Erfassen der vom gasförmigen Quecksilber ausgehenden radioaktiven γ-Strahlung aufweist. Die Messstrecke 5 wiederum ist an eine Kühleinrichtung 6 angeschlossen, in der das gasförmige Quecksilber kondensiert und in die Sammeleinrichtung 7 überführt wird.

Zusätzlich zu der auf den Vorlagebehälter 2 wirkenden Heizeinrichtung 4 ist eine auf die Messstrecke 5 wirkende weitere Heizeinrichtung 4 vorgesehen, die verhindert, dass die Quecksilberprobe in der Messstrecke 5 kondensiert.

Schließlich sind eine erste Waage 3 zur Bestimmung der Masse der im Vorlagebehälter 2 befindlichen Quecksilberprobe und eine zweite Waage 3 zur Bestimmung der Masse der im Sammelbehälter 7 befindlichen Quecksilberprobe vorgesehen.

Die Vorrichtung wird bevorzugt derart verwendet, dass nach der Befüllung und dem Schließen des Vorlagebehälters 2 die Heizungen 4 eingeschaltet werden. Insbesondere wird die auf die Messstrecke wirkende Heizeinrichtung 3 zuerst eingeschaltet, bis die erforderliche Betriebstemperatur erreicht ist - erst danach wird die auf den Vorlagebehälter 2 wirkende Heizung 4 eingeschaltet. Vorteilhaft wird ein Vakuum angelegt.

Nach Erreichen des Siedepunktes verdampft die Quecksilberprobe und der Quecksilberdampf strömt aus dem Vorlagebehälter 2 durch die beheizte Messstrecke 5 in die Kühleinrichtung 6, in der der Quecksilberdampf kondensiert. Von dort gelangt das Quecksilber in den Sammelbehälter 7 und kann nach Ende der Messung entnommen und der Entsorgung bzw. Aufbereitung zugeführt werden.

### Bezugszeichenliste

- 1: Vorratsbehälter
- 2: Vorlagebehälter
- 3: Waage
- 4: Heizeinrichtung
- 5: Messstrecke
- 6: Kühleinrichtung
- 7: Sammelbehälter

## Patentansprüche

1. Verfahren zur radiologischen Charakterisierung des Strahlungsinventars von Quecksilber,
mit den Schritten:
a. Bereitstellen einer vorbestimmten Menge Quecksilber,
b. Verdampfen wenigstens einer Teilmenge des bereitgestellten Quecksilbers, und
c. Erfassen der vom gasförmigen Quecksilber ausgehenden radioaktiven γ-Strahlung.

2. Verfahren nach Anspruch 1, **gekennzeichnet durch** die weiteren Schritte:
d. Kondensieren des gasförmigen Quecksilbers,
e. Auffangen des kondensierten Quecksilbers, und
f. Entsorgen des Quecksilbers in Abhängigkeit von der gemessenen radioaktiven γ-Strahlung.

3. Verfahren nach einem der vorhergehenden Ansprüche, **gekennzeichnet durch** Wiegen der bereitgestellten Menge Quecksilber und/oder der für das Verdampfen vorgesehenen Teilmenge des bereitgestellten Quecksilbers und/oder des aufgefangenen kondensierten Quecksilbers.

4. Verfahren nach Anspruch 3, **gekennzeichnet durch** Berechnen der gemessenen radioaktiven γ-Strahlung bezogen auf das Gewicht der bereitgestellten Menge Quecksilber und/oder der für das Verdampfen vorgesehenen Teilmenge des bereitgestellten Quecksilbers und/oder des aufgefangenen kondensierten Quecksilbers.

5. Verfahren nach einem der vorhergehenden Ansprüche, **dadurch gekennzeichnet, dass** das Erfassen der radioaktiven Strahlung das gammaspektroskopische Messen der radioaktiven Strahlung beinhaltet.

6. Verfahren nach einem der vorhergehenden Ansprüche, **gekennzeichnet durch** Bestimmen der im Quecksilber enthaltenen Radionuklide mittels des erfassten Gammaspektrums.

7. Verfahren nach Anspruch 6, **dadurch gekennzeichnet, dass** das Entsorgen des Quecksilbers in Abhängigkeit von der gemessenen radioaktiven γ-Strahlung und der Art der bestimmten, im Quecksilber enthaltenen Radionuklide erfolgt.

8. Vorrichtung zur radiologischen Charakterisierung des Strahlungsinventars von Quecksilber, mit
- einem Vorlagebehälter (2) zur Aufnahme einer zu untersuchenden Quecksilberprobe,
- einer auf den Vorlagebehälter (2) wirkenden Heizeinrichtung (4) zum Verdampfen der Quecksilberprobe,
- einer mit dem Vorlagebehälter (2) verbundenen Messstrecke (5), die ein Gammaspetkrometer zum Erfassen der vom gasförmigen Quecksilber ausgehenden radioaktiven γ-Strahlung aufweist, und
- einem mit der Messstrecke (5) verbundenen Sammelbehälter (7) zum Sammeln der die Messstrecke (5) durchlaufenen Quecksilberprobe.

9. Vorrichtung nach Anspruch 8, **gekennzeichnet durch** eine auf die Messstrecke (5) wirkende weitere Heizeinrichtung (4).

10. Vorrichtung nach einem der Ansprüche 8 und 9, **gekennzeichnet durch** eine zwischen Messstrecke (5) und Sammelbehälter (7) angeordnete Kühleinrichtung (6) zum Kondensieren des gasförmigen Quecksilbers.

11. Vorrichtung nach einem der Ansprüche 8 bis 10, **gekennzeichnet durch** eine erste Waage (3) zur Bestimmung der Masse der im Vorlagebehälter (2) befindlichen Quecksilberprobe und/oder eine zweite Waage (3) zur Bestimmung der Masse der im Sammelbehälter (7) befindlichen Quecksilberprobe.

12. Vorrichtung nach einem der Ansprüche 8 bis 11, **dadurch gekennzeichnet, dass** die Vorrichtung vakuum- und druckdicht ausgeführt ist.

## Claims

1. Method for the radiological characterization of the radiation inventory of mercury,
comprising the steps of:
a. providing a predetermined amount of mercury,
b. evaporating at least a portion of the provided mercury, and
c. detecting the radioactive γ-radiation emitted by the gaseous mercury.

2. Method according to claim 1, **characterized by** the further steps:
d. condensing the gaseous mercury,
e. collecting the condensed mercury, and
f. disposing of the mercury as a function of the measured radioactive γ-radiation.

3. Method according to one of the preceding claims, **characterized by** weighing the provided amount of mercury and/or the portion of the provided mercury intended for evaporation and/or the collected condensed mercury.

4. Method according to claim 3, **characterized by** calculating the measured radioactive γ-radiation based on the weight of the provided amount of mercury and/or the portion of the provided mercury intended for evaporation and/or the collected condensed mercury.

5. Method according to one of the preceding claims, **characterized in that** detecting the radioactive radiation includes the gamma spectroscopy measurement of the radioactive radiation.

6. Method according to one of the preceding claims, **characterized by** determining, by means of the detected gamma spectrum, the radionuclides contained in the mercury.

7. Method according to claim 6, **characterized in that** the disposal of the mercury takes place as a function of the measured radioactive γ-radiation and the type of the determined radionuclides contained in the mercury.

8. Apparatus for the radiological characterization of the radiation inventory of mercury, comprising
- a storage container (2) for receiving a mercury sample to be examined,
- a heating device (4), acting on the storage container (2), for evaporating the mercury sample,
- a measurement section (5) which is connected to the storage container (2) and has a gamma spectrometer for detecting the radioactive γ-radiation emitted by the gaseous mercury, and
- a collecting container (7), connected to the measurement section (5), for collecting the mercury sample passing through the measurement section (5).

9. Apparatus according to claim 8, **characterized by** a further heating device (4) acting on the measurement section (5).

10. Apparatus according to one of claims 8 and 9, **characterized by** a cooling device (6), arranged between measurement section (5) and collecting container (7), for condensing the gaseous mercury.

11. Apparatus according to one of claims 8 to 10, **characterized by** a first scale (3) for determining the mass of the mercury sample located in the storage container (2) and/or a second scale (3) for determining the mass of the mercury sample located in the collection container (7).

12. Apparatus according to one of claims 8 to 11, **characterized in that** the apparatus is designed to be vacuum-tight and pressure-tight.

## Revendications

1. Procédé permettant la caractérisation radiologique de l'inventaire de rayonnement de mercure,
comportant les étapes de :
a. fourniture d'une quantité prédéterminée de mercure,
b. évaporation d'au moins une quantité partielle du mercure fourni, et
c. détection du rayonnement γ radioactif émis par le mercure gazeux.

2. Procédé selon la revendication 1, **caractérisé par** les étapes supplémentaires de :
d. condensation du mercure gazeux,
e. collecte du mercure condensé, et
f. élimination du mercure en fonction du rayonnement γ radioactif mesuré.

3. Procédé selon l'une des revendications précédentes, **caractérisé par** le pesage de la quantité de mercure fournie et/ou de la quantité partielle du mercure fourni prévue pour l'évaporation et/ou du mercure condensé collecté.

4. Procédé selon la revendication 3, **caractérisé par** le calcul du rayonnement γ radioactif mesuré par rapport au poids de la quantité de mercure fournie et/ou de la quantité partielle du mercure fourni prévue pour l'évaporation et/ou du mercure condensé collecté.

5. Procédé selon l'une des revendications précédentes, **caractérisé en ce que** la détection du rayonnement radioactif comprend la mesure par spectroscopie gamma du rayonnement radioactif.

6. Procédé selon l'une des revendications précédentes, **caractérisé par** la détermination des radionucléides contenus dans le mercure au moyen du spectre gamma détecté.

7. Procédé selon la revendication 6, **caractérisé en ce que** l'élimination du mercure est effectuée en fonction du rayonnement γ radioactif mesuré et du type de radionucléides déterminés contenus dans le mercure.

8. Dispositif permettant la caractérisation radiologique de l'inventaire de rayonnement de mercure, comportant
- un récipient de stockage (2) destiné à recevoir un échantillon de mercure à analyser,
- un appareil de chauffage (4) agissant sur le réservoir de stockage (2) pour l'évaporation de l'échantillon de mercure,
- une section de mesure (5) reliée au récipient de stockage (2) et présentant un spectromètre gamma pour la détection du rayonnement γ radioactif émis par le mercure gazeux, et
- un récipient de récolte (7) relié à la section de mesure (5) et permettant de récolter l'échantillon de mercure traversant la section de mesure (5).

9. Dispositif selon la revendication 8, **caractérisé par** un autre appareil de chauffage (4) agissant sur la section de mesure (5).

10. Dispositif selon l'une des revendications 8 et 9, **caractérisé par** un appareil de refroidissement (6) disposé entre la section de mesure (5) et le récipient de récolte (7) pour la condensation du mercure gazeux.

11. Dispositif selon l'une des revendications 8 à 10, **caractérisé par** une première balance (3) pour la détermination de la masse de l'échantillon de mercure se trouvant dans le récipient de stockage (2) et/ou une seconde balance (3) pour la détermination de la masse de l'échantillon de mercure se trouvant dans le récipient de récolte (7).

12. Dispositif selon l'une des revendications 8 à 11, **caractérisé en ce que** le dispositif est étanche au vide et à la pression.
